# EUROPEAN PATENT SPECIFICATION

(11) **EP 1 557 192 B1**
(45) Date of publication and mention of the grant of the patent: **25.08.2010**
(21) Application number: 05250337.2
(22) Date of filing: 24.01.2005
(51) Int. Cl.: A61M 25/01, A61M 25/00

(54) **Catheter assembly with two stylets**
Katheteranordnung mit zwei Stiletts
Ensemble de catheter avec deux stylets

(30) Priority: 23.01.2004 US 764674
(43) Date of publication of application: 27.07.2005
(73) Proprietor: Quinn, David G., Grayslake, IL 60030 (US)
(72) Inventor: Quinn, David G., Grayslake, IL 60030 (US)
(74) Representative: McLeish, Nicholas Alistair Maxwell

(56) References cited:
- WO-A-94/28953
- WO-A-99/44667
- WO-A-03/043679
- WO-A-03/061752
- WO-A-2004/067078
- DE-A1- 4 320 186
- US-A- 4 548 206
- US-A- 5 382 238
- US-A- 5 902 285

## Description

### FIELD OF THE INVENTION

This invention relates generally to catheters and catheter insertion methods. It relates particularly to catheter, stylet and guide wire assemblies for introducing a catheter into a patient's body cavity. It also relates to methods of inserting catheters into a patient's jejunum. A catheter as defined in the preamble of claim 1 is disclosed in WO 03/061752.

### BACKGROUND OF THE INVENTION

Stylets are used in medical practice situations where the clinician desires to push the catheter into a body cavity or vessel. Naso-enteral feeding tubes are inserted with stylets, as are Groshong intravenous catheters. Stylets have constant stiffness over their entire length, however. This can be a disadvantage when the catheter must travel around acute angle bends or through valves or sphincters. Having the stylet constructed with a softer leading portion can be a disadvantage because in its course of travel the catheter may encounter obstructions that will not readily permit the entry of the leading softer portion of the stylet.

Guide wires, on the other hand, are used where the clinician simply wishes to guide a catheter into a body cavity. A naso-enteral feeding tube, for example, might be guided into a patient's stomach over a guide wire.

Neither presently known stylet or guide wire assemblies or systems are effectively utilized where jejunal insertion is desired. This is particularly problematical at the present time because there is an increasing interest in placing naso-enteral feeding tubes into the jejunum, rather than the stomach or duodenum. Clinical data shows that in long term(one week or more) nasal-enteral feeding the incidence of aspiration and reflux of feeding formula into the lungs is more than 20%. This reflux occurs because the duodenum has weak peristalsis and is subject to retrograde (reverse) peristalsis that rends to push feeding formula and/or feeding tubes back into the stomach. On the other hand the jejunum has strong peristalsis. This strong peristalsis coupled with the added bends of the tube in the intestine aids in keeping the tube in place in the jejunum and keeping the formula isolated from the stomach.

There is also an increased understanding of the need for immediate jejunal feeding after gastric or intestinal surgery. Present practice is for the patient to be denied any oral, nasogastric or naso-jejunal feeding post surgically until peristalsis returns. It takes between one and eight days for peritalsis to return depending on the degree of gastric or intestinal insult caused by the surgery. During this period a suction tube is placed into the stomach via the nasal route to aspirate any gastric juices that build up in the stomach and present the danger of pulmonary aspiration.

Present practice is to place tubes into the jejunum by endoscopy. Patient sedation is required and gastroenterologists have difficulty pushing the tubes out of the stomach, through the pylorus and then past the Ligament of Trietz.

### SUMMARY OF INVENTION

An object of the invention is to provide a new and improved catheter and stylet, guidewire assembly for inserting a feeding tube into a body cavity.

Another object is to provide a catheter and stylet guidewire assembly affording almost infinite varying stiffness-adjustability over its leading distal portion.

Still another object is to provide a catheter and, guidewire assembly with variable stiffness over much of the entire length.

A further object is to provide a catheter and combination stylet and guide wire assembly for inserting a feeding tube into a body cavity.

Yet a further object is to allow the insertion of a naso-enteral tube into the jejunum by pushing the tube exteriorly, thereby eliminating the need for endoscopy.

Another object is to provide a tube insertion assembly which allows the use of a small, very flexible 8Fr feeding tube, rather than stiffer 10Fr or 12for tubes.

Still another object is to provide a catheter stylet and guide wire assembly which facilitates easy insertion of the catheter into the jejunum yet prevents, through the catheter design, undesirable withdrawal of the catheter therefrom.

Another object is to prevent coiling of the tube in the stomach during the procedure of inserting it through the pylorus.

Another objective is to provide a catheter tube that creates minimal friction along its entire outside surface from the nares to its tip in the jejunum.

Another object is to prevent necrosis in the nasopharynx, pylorus and the Ligament of Trietz by allowing the use of a small, flexible 8Fr tube.

Another object is to incorporate a stylet tip configuration that provides maximum protection against the inadvertent pushing of the stylet through the tube wall.

Another object is to provide a catheter with very lubricious water activated internal and external coating to assure both easy insertion and free movement of the stylet and guide wire within the tube.

Still another object is to provide a catheter, stylet and guide wire assembly of the aforedescribed character which includes a multi-lumen tube containing a gastric suction lumen.
According to the present invention there is provided a catheter insertion assembly according to the appended claims.

The catheter and stylet and guide wire assembly of the present invention can be used for any medical catheter insertion where a stylet and guide wire is conventionally used. One embodiment described in the present application is used for naso-enteral insertion of a feeding tube into and through the stomach, the duodenum and finally into the jejunum. This use presents some unique requirements for adjustable variability of tube stiffness. Moderate stiffness is provided for initial insertion through the nasopharynx, the esophagus and into the stomach. Considerably more stiffness is then provided to transmit pushing force from outside the patient to the catheter tip as it is pushed through the pylorus into the duodenum and to the Ligament of Trietz. After the stiffened leading end of the catheter tube reaches the Ligament of Trietz, it is made more flexible so it can navigate around the tight curve formed by the Ligament of Trietz and into the jejunum.

In this jejunal insertion application, the stylet assembly of the invention provides moderate stiffness over a long, 63.5 - 88.9 cm (25-35 inch) distal length during gastric insertion. Much greater stiffness is then provided over the entire length of the catheter during duodenal insertion. Finally, moderate to very little stiffness is provided over 25.4 to 38.1 cm (10 to 15 inches) of the distal end as it enters the jejunum.

In one embodiment which does not show the invention, an assembly has two stylets and allows the stylet wire of one to pass through the proximal connector of the other.

This assembly also allows irrigation of the inside of the tube through the stylet connection of the assembly to activate a lubricious coating inside the tube lumen (which allows stylet wire manipulation and removal). The coating is also on the exterior surface of approximately the first 88.9 cm (35 inches) of the distal tube and tip to minimize friction along the surface of the tube from the insertion point at the nares along its entire length to the tip in the jejunum. The coating adheres to the urethane surface and is activated by contact with water.

The stylet that seats itself in the feeding tube Y-connector contains a stylet wire whose tip end position is most distal. This stylet is referred to as the primary stylet. A secondary stylet which does not fall in the scope of claims 1-7 connects to the luer lock of the primary stylet and its tip is normally immediately adjacent the primary stylet tip.

The overall tube is approximately 152.4 cm (60 inches long). This compares to a conventional feeding tube length of approximately 114.3 cm (45 inches). The extra length tube provides for entry into the jejunum.

A catheter tube using a stylet and guide wire assembly may be inserted as follows:

1. inserting a stylet and a stylet connector into a Y-connector on the proximal end of a feeding tube; and connecting the stylet connector to the Y-connector;

2. flushing the tube with water through the connector to activate the lubricious coating on the entire length of the tube's internal lumen;

3. dipping approximately 15.24 cm (6 inches) of the tube and tip in water;

4. feeding the tube containing the stylet into the stomach, while moistening the exterior of the tube to activate the external lubricant;

5. inserting a guide wire through the stylet connector and feeding tube until its tip reaches the distal end of the tube, adjacent the stylet tip;

6. under fluoroscopy, advancing the guide wire through the pylorus and the duodenum, past the Ligament of Trietz and into the jejunum;

7. advancing the stylet stiffened tube through the pylorus and the duodenum, to the Ligament of Treitz;

8. pulling the stylet tip back through the pylorus;

9. under fluoroscopy, advancing the catheter tip on the wire, past the Ligament of Trietz, into the jejunum; and

10. while holding the Y-connector, retracting and discarding the wire and stylet.
In an alternate to this approach, the tube may be advanced from the stomach through the pylorus over the guide wire alone, i.e., without advancing the stylet further.

In an alternate form of the invention assemblies and methods, a multi-lumen tube with a mid-port bolus is employed. The catheter contains a suction lumen, in addition to the feeding lumen, and accesses both the stomach and the jejunum. The stylet and guide wire systems of the invention can also be used in this multi-lumen configuration. They then provide for both jejunal feeding and gastric suction.

Regardless of whether the aforedescribed assemblies and methods incorporate single lumen or multi-lumen tubes with mid-port boluses, or utilize dual stylet or stylet and guide wire combinations, the invention includes a normally coiled, single lumen tube section formed in the tube adjacent the distal end of the tube. This coiled section is then uncoiled, i.e. straightened, by a stylet and/or the guide wire and passed through the stomach, pylorus and duodenum to the Ligament of Trietz. The tip of the tube is then moved into the jejunum over the guide wire in this configuration. When the bolus tip of the tube is property positioned in the jenunum, the guide wire is removed, permitting the section to resume its normally coiled, larger diameter configuration. This configuration enhances the effect of peristalsis in the jejunum on the tube and tip, and retains them more securely in the jejunum.

### BRIEF DESCRIPTION OF THE DRAWING

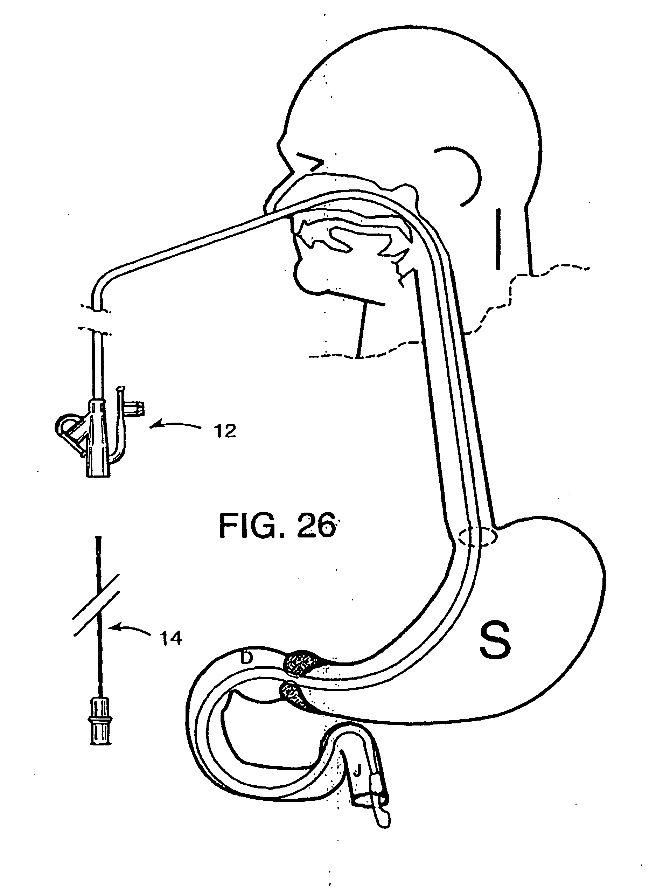

The invention, including its construction and method of operation, is illustrated more or less diagrammatically in the drawings of fig. 1-9, 25, 26, 30-39. However fig. 10-24, 27-29 do not fall in the scope of claims 1-7.

FIG. 1 is a side elevational view of a common, flow-through stylet connector;

FIG. 2 is an end view of the connector of FIG. 1;

FIG. 3 is a sectional view through the connector of FIG. 1 showing the flow-through lumen and the socket for the wire stylet;

FIG. 4 is a sectional view of the connector of FIG. 1 with a stylet in place;

FIG. 5 is a side elevational view of the assembled primary stylet and connector;

FIG. 6 is a side elevational view of a short segment of vinyl tubing;

FIG. 7 is a side elevational view of a male luer lock connector designed to fit over a delivery tube;

FIG. 8 is a side elevational view of the flow-through connector shown in FIG. 5, assembled to the male luer lock in FIG. 7 by means of both parts being inserted and glued into the short segment of vinyl tubing;

FIG. 9 is a longitudinal cross sectional view of the assembly of FIG. 8;

FIG. 10 is a side elevational view of the primary and secondary stylets connected, the tip of the secondary stylet being slightly behind the tip of the primary stylet;

FIG. 11 is a longitudinal cross sectional view of the primary and secondary stylets unconnected, but with the secondary stylet extending through the flow-through lumen of the primary stylet;

FIG. 12 shows the distal ends of both stylets' as they would be positioned in a feeding tube with both stylet connectors attached;

FIG. 13 is a sectional view through the tube in FIG. 12, at line 13-13 of FIG. 12, showing the main body of the primary stylet and through the tip of the secondary stylet, the tube being 8Fr with an 0.203 cm (0.080 inches) internal lumen and the stylet wire having a 0.036 cm (0.014 inch) OD;

FIG. 14 is a sectional view through the tube in FIG. 12 and the main bodies of both stylets, at line 14-14;

FIG. 15 is a side elevational view of the first form of catheter assembly, with the stylet connectors assembled, but not seated, in the feeding tube Y-connector with a cross sectional view of the tube and tip showing the position of the stylets;

FIG. 16 is a view similar to FIG. 15 showing the connected two stylet assembly seated in the Y-connector with the distal tip of the primary stylet positioned just behind the tube tip;

FIG. 17 is a view similar to FIG. 15 showing the two stylet assembly with the secondary stylet retracted;

FIG. 18 is a view similar to FIG. 15 showing both stylets disconnected from each other and from the Y-connector;

FIG. 19 is a view similar to FIG. 15 showing the secondary stylet removed and the primary stylet seated in the Y-connector as it would be in a normal feeding tube insertion procedure;

FIG. 20 is a longitudinal sectional view through a tube illustrating the almost infinite stiffness adjustability of the catheter, i.e., there are three potential stiffness zones in the tube, all adjustable;

FIG. 21 shows a feeding tube during insertion in a patient with the secondary stylet retracted approximately 35 inches so that gastric insertion can be carried out with normal stylet stiffness;

FIG. 22 shows the secondary stylet re-seated in the Y-connector;

FIG. 23 shows both stylets remaining in place for maximum stiffness, the catheter having been pushed through the pylorus and into the duodenum up to the Ligament of Trietz;

FIG. 24 shows the secondary stylet retracted approximately 30.48 cm (12 inches), the primary stylet remaining in place, so as to provide reduced but adequate stiffness so that the tube can be pushed around the Ligament of Trietz into the jejunum;

FIG. 25 shows the secondary stylet removed after final placement of the tip in the jejunum;

FIG. 26 illustrates the tube ready for infusion of nutrients after careful removal of the secondary stylet;

FIG. 27 is a side elevational view of a mid-port bolus in a multi-lumen feeding tube with the dual stylet embodiment of the invention are employed;

FIG. 28 is a top plan view of the bolus of FIG. 27;

FIG. 29 is a longitudinal sectional view taken along lines 29-29 of FIG. 27;

FIG. 30 shows a feeding tube with a stylet assembly that has been inserted into the stomach with the tube tip positioned adjacent to the pylorus;

FIG. 31 shows the stylet fitting seated in the Y connector and the distal end of the stylet immediately adjacent the bolus tip. The guide wire has been fed through the catheter assembly into the jejunum while the catheter tube and tip remain adjacent to the pylorus in the stomach;

FIG. 32 shows the catheter tube and tip after it has been pushed through the pyloric valve over the guidewire and is adjacent to the Ligament of Treitz, still on the guidewire;

FIG. 33 shows the catheter tube and tip after the stylet has been released from the Y connector and the tube has been advanced over the guide wire to its final position beyond the Ligament of Treitz;

FIG. 34 shows the catheter tube with the stylet removed;

FIG. 35 shows the catheter tube with the guide wire pulled out completely;

FIG. 36 shows a cross-section of the tip bolus with a passage for the guide wire;

FIG. 37 is a side elevational view of the distal end of a catheter tube and tip of the present invention, the tube incorporating a coiled section adjacent the bolus tip;

FIG. 38 is a view similar to FIG. 35 showing the tube at its distal end threaded over a guide wire whereby the coil section of the tube has been uncoiled by the stiffer wire, and a stylet inserted to a point adjacent the bolus tip; and

FIG. 39 is an enlarged view of the distal end of the tube with the bolus tip in the jejunum and the coiled section recoiled after removal of the guide wire and stylet.

### DESCRIPTION OF THE PREFERRED EMBODIMENTS

Referring to the drawings, a catheter and stylet assembly embodying features of the invention is shown generally at 10. The assembly 10 comprises a catheter tube sub-assembly 12, a first stylet sub-assembly 14 and a second stylet sub-assembly 16.

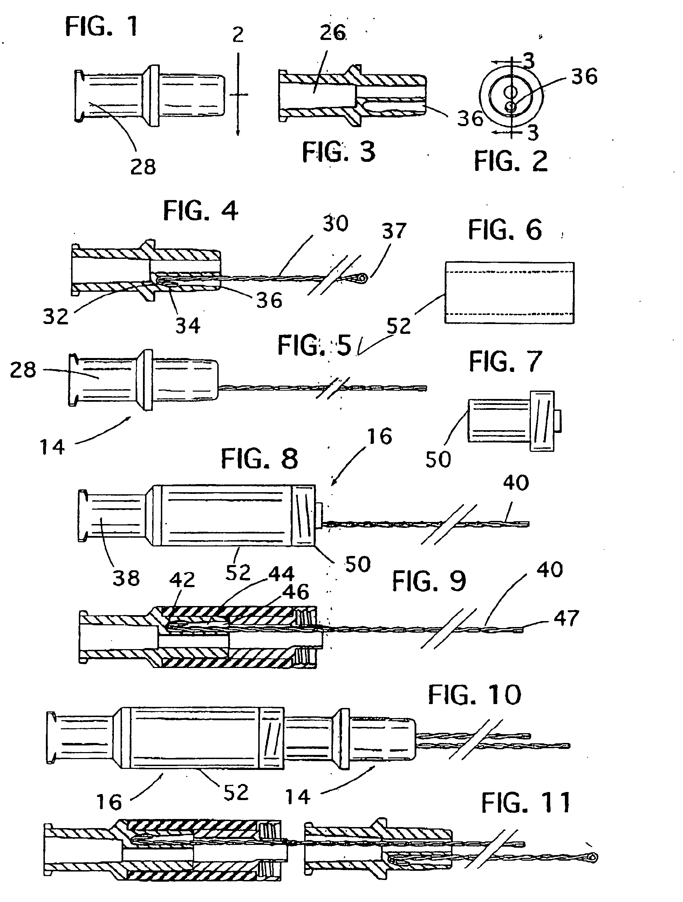

Referring now also to FIGS. 1-9, the catheter tube sub-assembly 12 comprises an 8 French (Fr) tube 20 having a bolus tip 22 on its distal end. A conventional Y-connector 24 is mounted on its proximal end. The Y-connector has a conventional inlet port 26 at its proximal end.

The first stylet sub-assembly 14 comprises a molded acrylic connector sleeve 28 and a primary twisted wire stylet 30. The proximal end 32 of the stylet 30 is force fit into a slot 34 formed within, and on the side, of a lumen 36 extending longitudinally through the connector sleeve 28. The distal end 37 of the stylet extends approximately 121.92 cm (48 inches) from the nose of the sleeve 28.

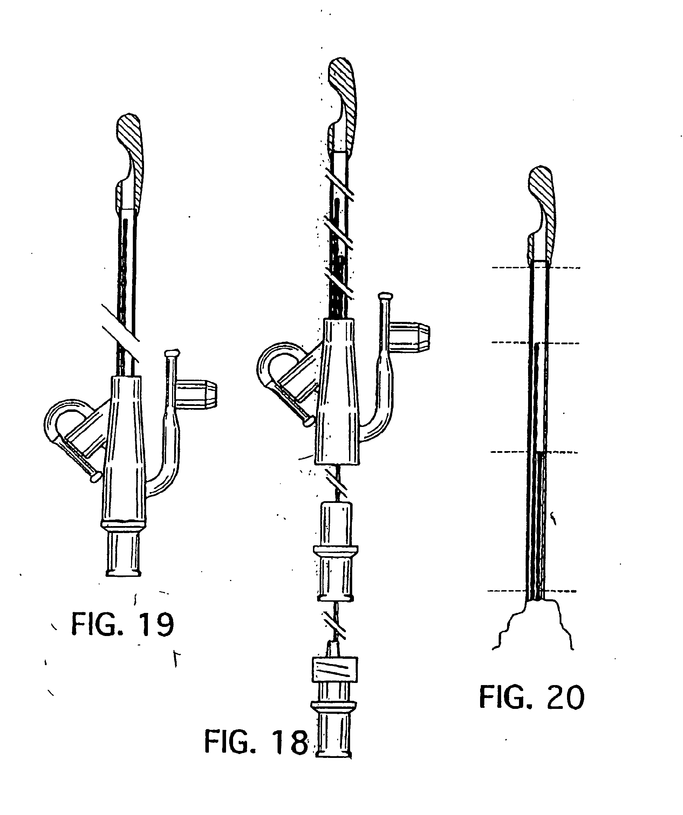

The connector sleeve 28 can be threaded into the proximal end port 26 of the Y-connector 24. Its stylet 30 then extends through the tube 20, as illustrated in FIG. 19.

The second stylet sub-assembly 16 which does not fall in the scope of claims 1-7 comprises a molded acrylic connector sleeve 38 identical to the sleeve 28. A secondary twisted wire stylet 40 has a proximal end 42 which is force fit into a slot 44 formed inside, and on one side of, a lumen 46 extending longitudinally through the connector sleeve 38. The distal end 47 of the secondary stylet 40 extends approximately 132.08 cm (52 inches) from the nose of the sleeve 38.

The second stylet sub-assembly 16 also comprises an acrylic, male luer lock fitting 50 and a vinyl sleeve 52. The vinyl sleeve 52 connects the luer lock fitting 50 to the connector sleeve 38.

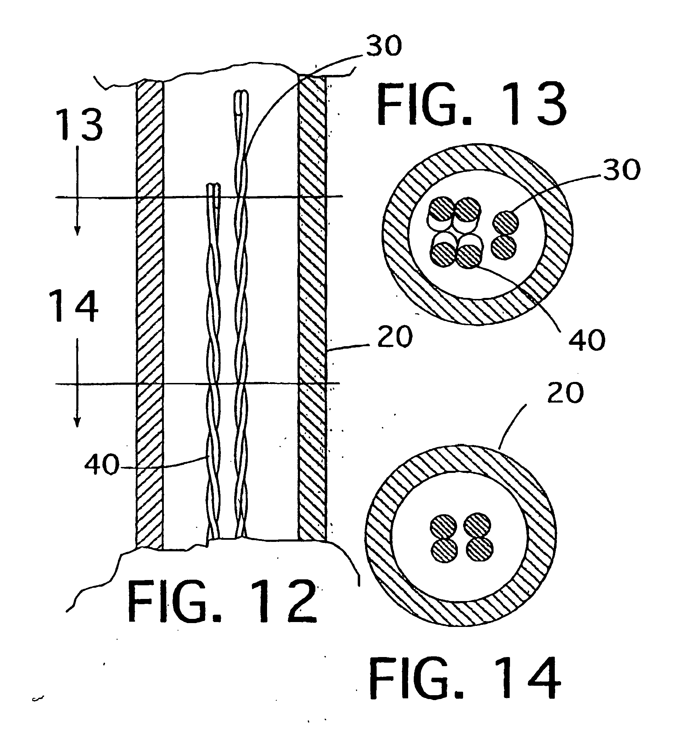

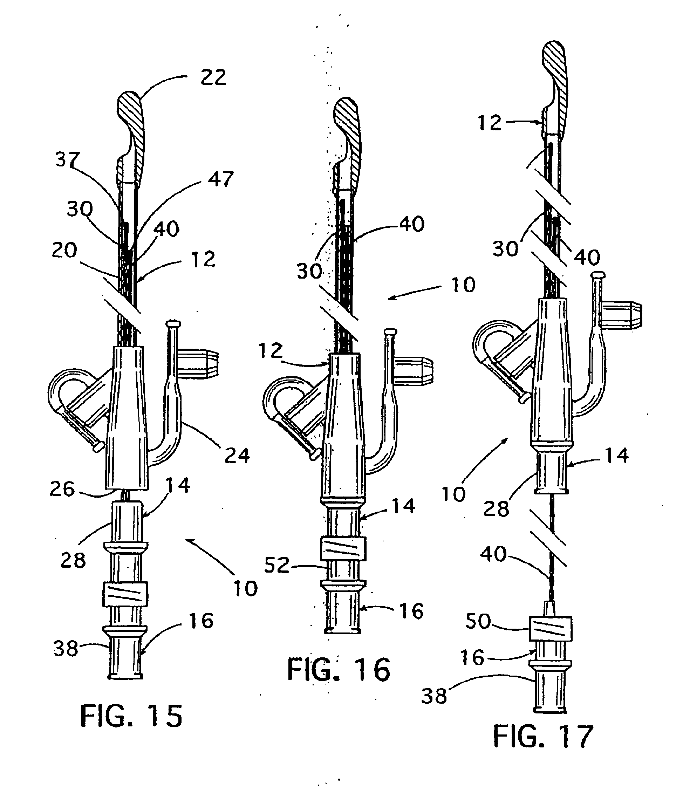

The catheter tube sub-assembly 12, first stylet sub-assembly 14 and second stylet sub-assembly 16 are joined together to form the assembly 10 in the manner illustrated in FIG. 16. In this assembly 10, the relationship of the stylets 30 and 40 are shown in FIGS. 12-14.

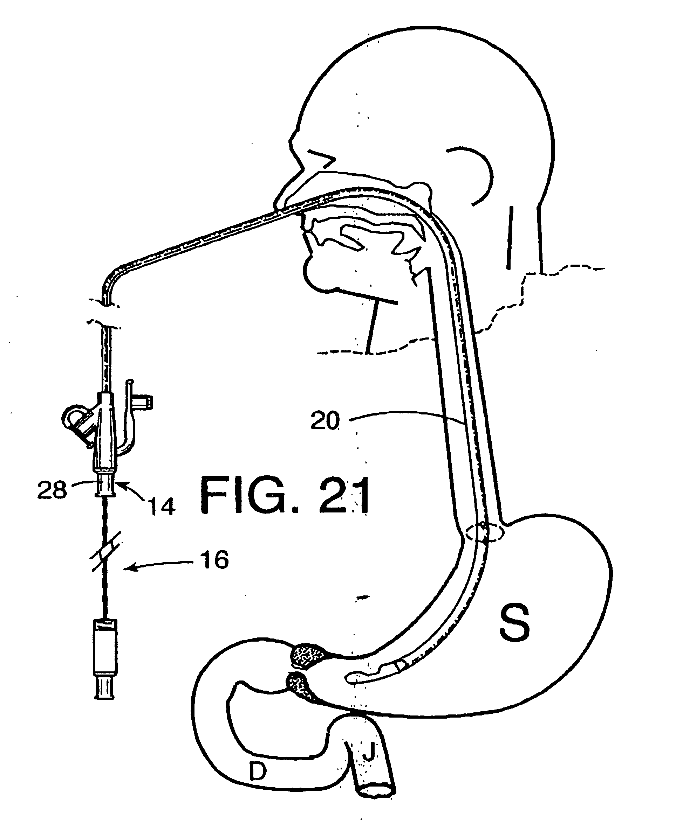

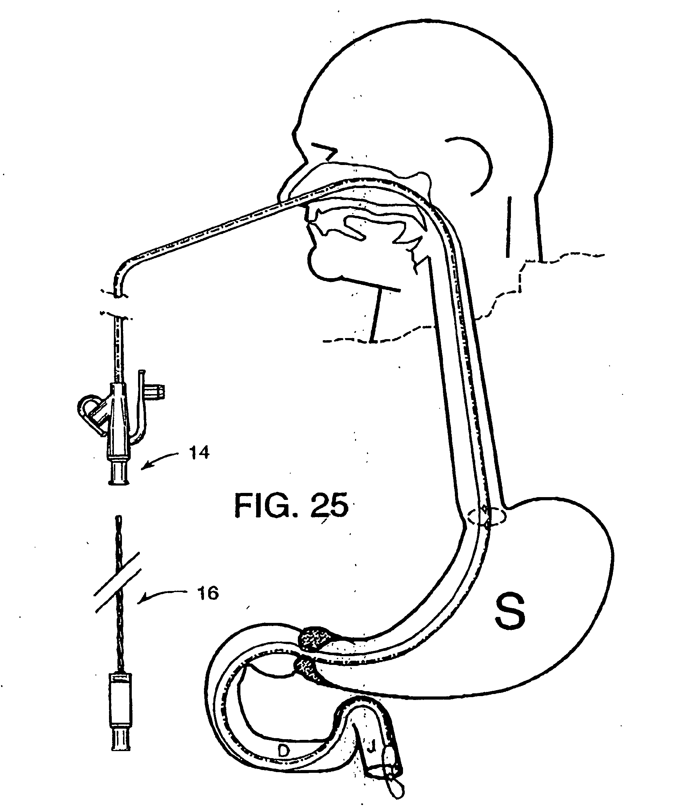

Referring now to FIGS. 21-25, FIG. 21 shows stylet sub-assembly 16 disconnected from stylet sub-assembly 14 and its stylet 28 withdrawn approximately 88.9 cm (35 inches). In this configuration, the assembly 10 provides a more flexible tube 20 for insertion through a patient's nose.

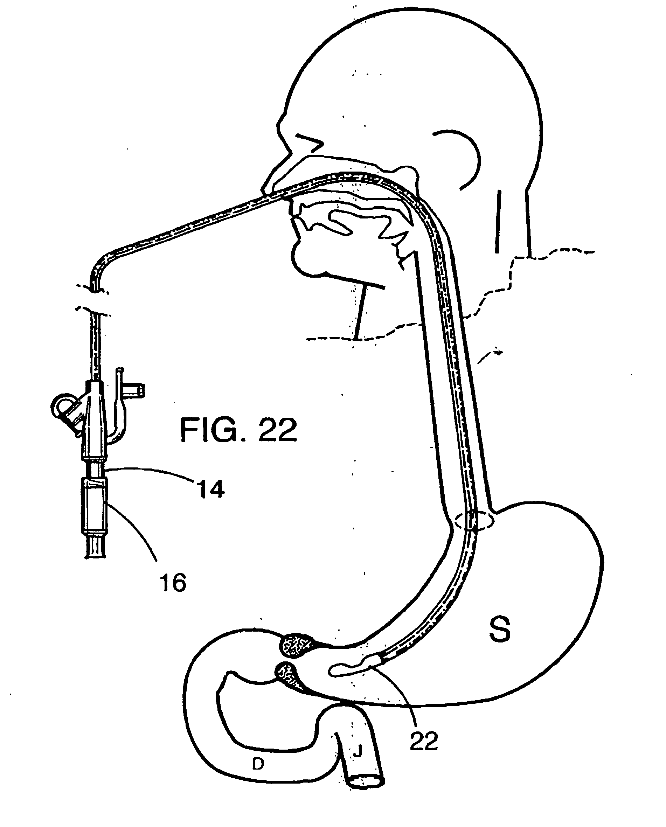

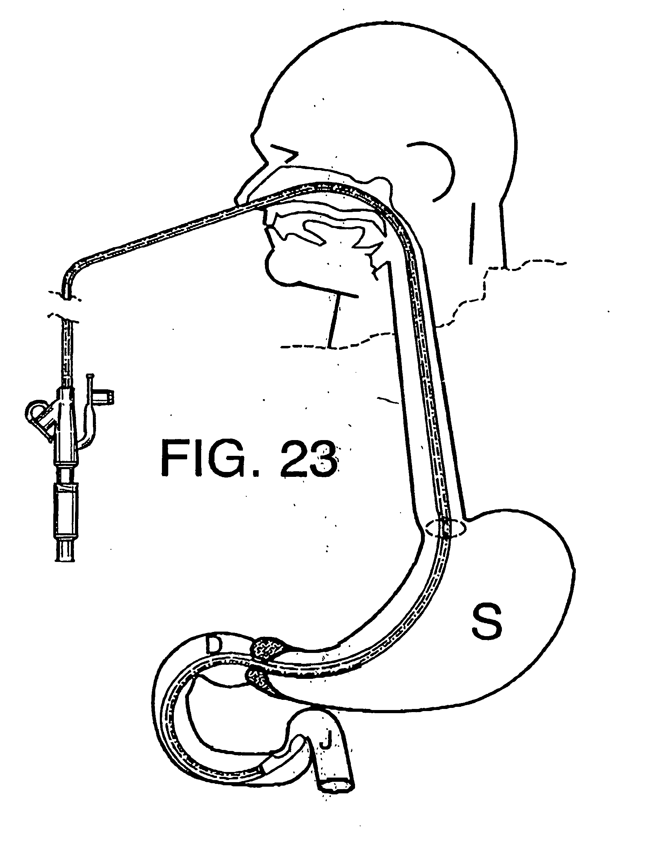

In FIG. 22, the first and second stylet sub-assemblies 14 and 16 are connected. In this configuration both stylet tips are positioned just behind tube tip 22. This facilitates passage of the tube 20 through the pyloric valve to the position shown in FIG. 23.

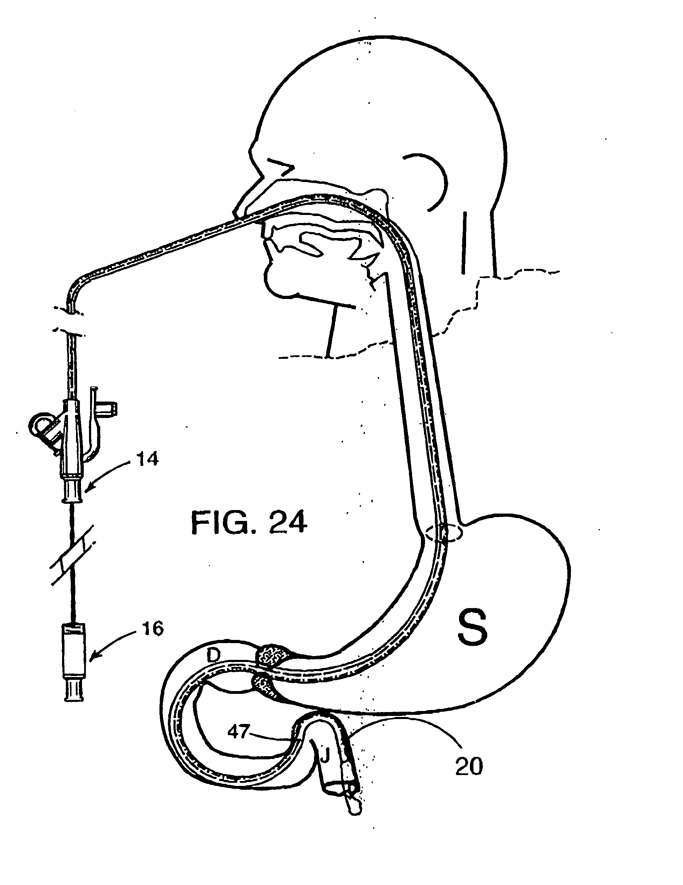

When a more flexible distal end of the tube 20 is needed, the second stylet sub-assembly 16 is disconnected from the first stylet sub-assembly 14 using the luer lock fitting. The secondary stylet can then be withdrawn a short distance (12 inches as seen in FIG. 24). This permits easy passage into the duodenum, for example.

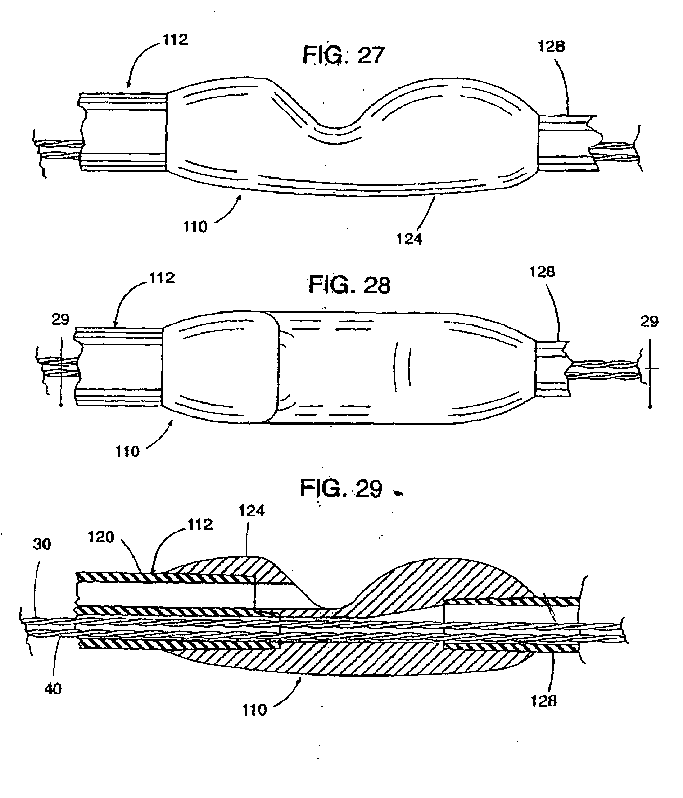

Referring now to FIGS. 27-29 which do not fall in the scope of claims 1-7, a modified application is illustrated in the form of a catheter and stylet assembly 110 (with parts removed). In the assembly 110, the primary and secondary stylets 30 and 40 extend through a dual lumen catheter tube sub-assembly 112. The catheter tube sub-assembly 112 includes an 8 Fr dual lumen tube 120, a mid-port bolus 124 and a 5 Fr single lumen tube 128. The stylets 30 and 40 extend through the lumens and the mid-port bolus in the manner illustrated.

The use of a lubricious coating in the catheter insertion process has previously been described. The lubricant is a combination of methyl cellulose and polyvinylpyrolidone. The lubricant adheres to the tube surface and becomes extremely slippery when contacted by water.

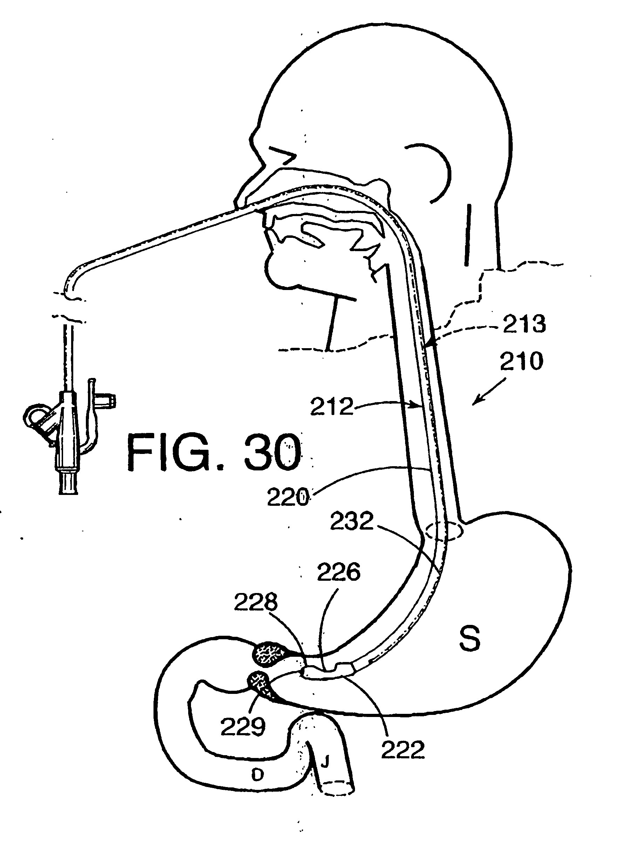

Turning now to FIGS. 30-35, a catheter, stylet and guide wire assembly embodying features the invention is shown generally at 210. The assembly 210 comprises a catheter tube sub-assembly 212, a stylet sub-assembly 213 and a guide wire 214. The catheter tube sub-assembly includes a catheter tube 220 and tip bolus 222.

There are several ways of inserting the catheter tip 222 into the jejunum. With a first, an ultra-slim endoscope is inserted through the nose and advanced through the stomach, into the duodenum, through the pyloric valve. The scope is advanced just proximal to the Ligament of Treitz. The guide wire 214 is then threaded through the scope and fed beyond the scope, around the Ligament of Trietz, into the jejunum. The scope is then carefully removed, leaving the guide wire in place. The catheter assembly with the stylet in place is then fed over the guide wire until the bolus tip 222 comes into proximity with the pyloric valve. The catheter tip 222 has a passage 229 formed in its bullet nose 228 that allows the passage over the guide wire. The stylet connector is then unseated from the catheter Y-connector. The stylet is held in place so that its distal end remains just proximal to the pylorus as the catheter is advanced over the guide wire until the bolus tip 222 is positioned beyond the Ligament of Treitz, in the jejunum. The guide wire is then removed and the catheter is ready for jejunal feeding.

Using a second procedure, the catheter is positioned in the stomach near the pylorus by using either a single stylet or the previously described dual stylet system In the case of the dual stylet system, the secondary stylet is removed prior to the insertion of the guidewire. After the guidewire is threaded into the jejunum, the insertion procedure is identical to the first method described above. This second procedure is sequentially illustrated in FIGS. 30-35.

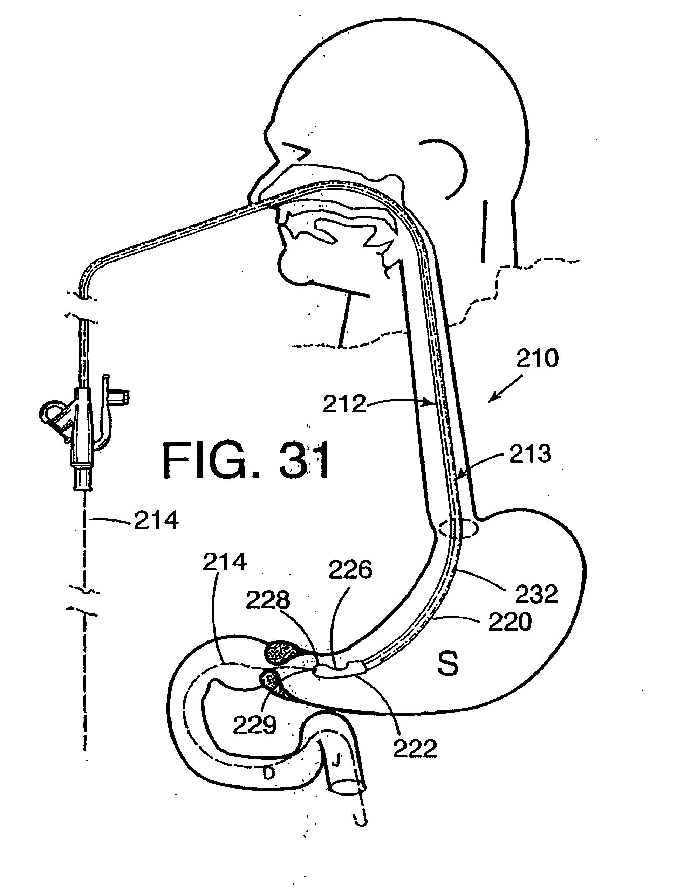

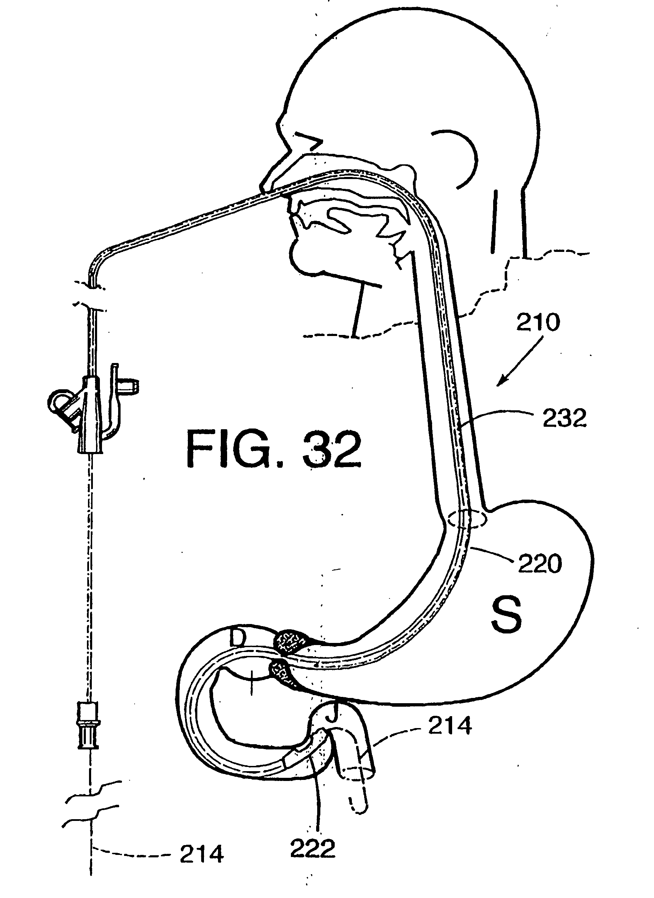

FIG. 30 shows the catheter assembly 212 in place with its tip bolus 222 next to the pylorus and a single stylet 282 in place immediately adjacent the tip bolus. FIG. 31 shows the guide wire 214 threaded through the catheter, into the duodenum and beyond the Ligament of Treitz. FIG. 32 shows the stylet connector disconnected from the catheter tube assembly and the catheter tube advanced over the guide wire 214 into the duodenum, up to a point just before the Ligament of Treitz. In the illustrated method, the distal end of the stylet remains in the stomach. However, the stylet might be advanced through the pylorus into the duodenum to provide additional stiffness to the catheter, as previously discussed.

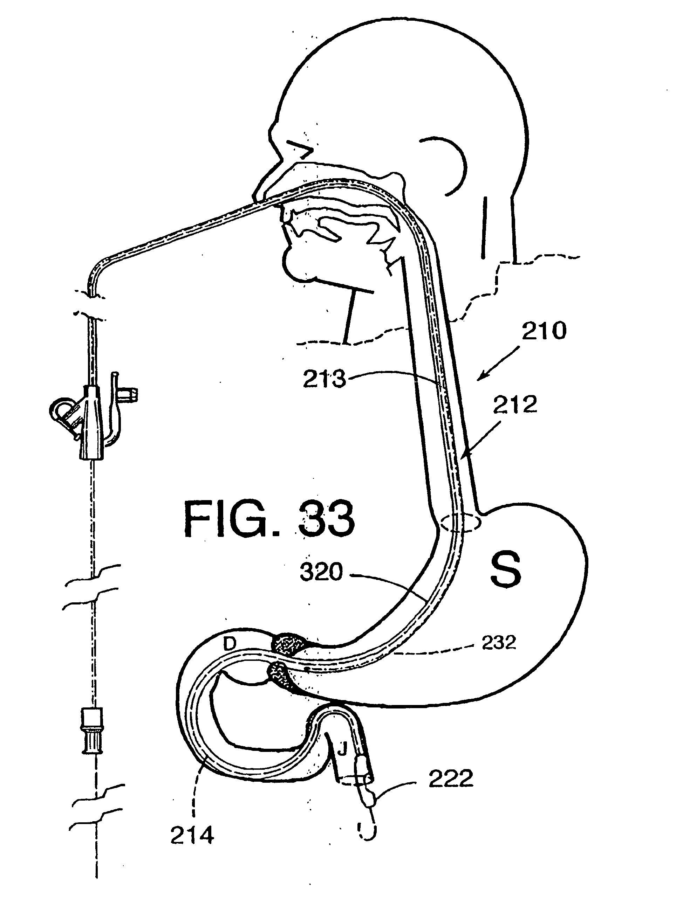

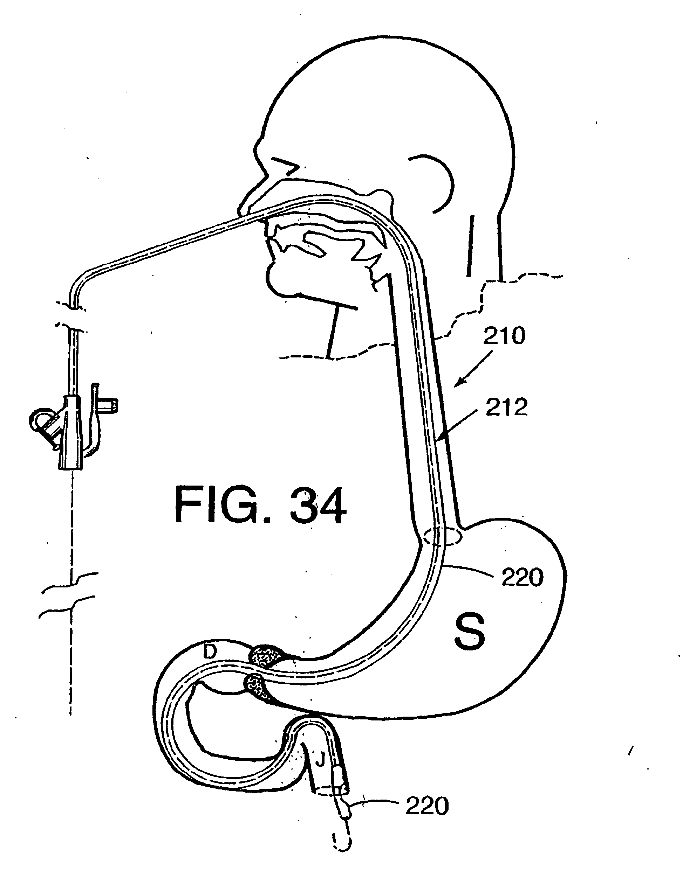

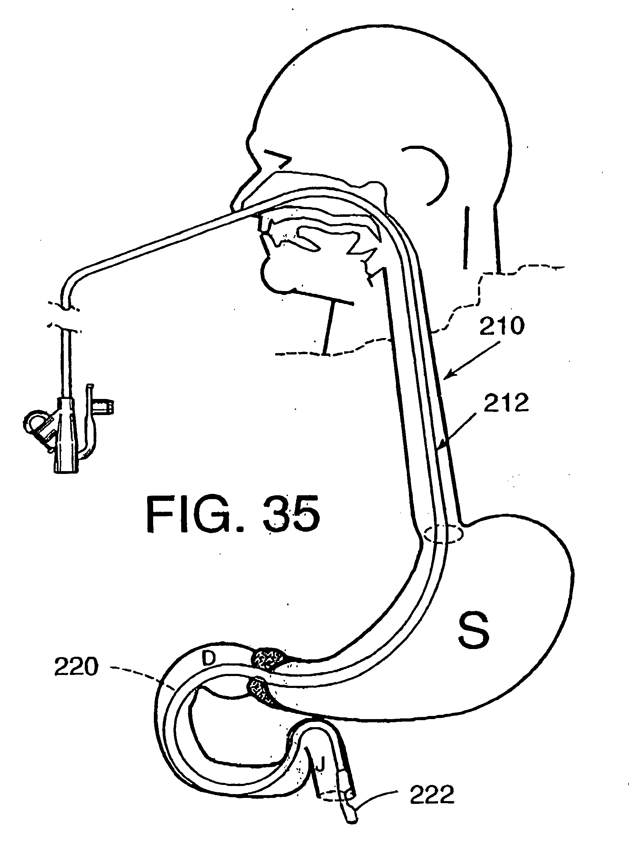

FIG. 33 then shows the catheter advanced to its final position, beyond the Ligament of Treitz and in the jejunum. FIG. 34 shows the removal of the stylet 232. FIG. 35 shows the removal of the guide wire 214. The catheter is then ready for feeding.

To this point the assembly and method have been described in the context of a standard polyurethane feeding tube with a bolus on its distal end. The invention however contemplates using a modified tube construction which enhances retention capabilities in the jejunum, however.

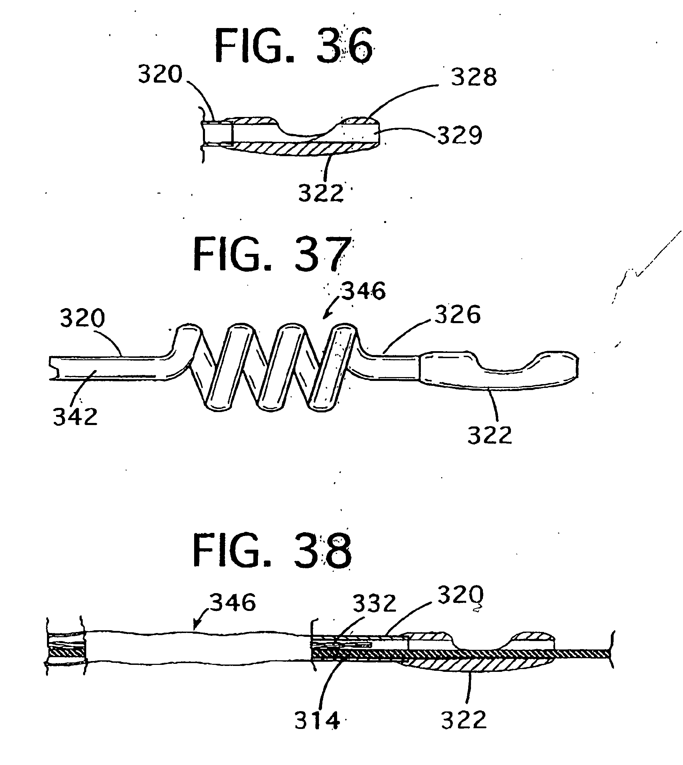

Referring to FIGS. 36-39, a catheter tube 320 is extruded to form the substantially linear section 342 extending from a conventional connector (not shown) at its proximal end to a short coil section 346 adjacent the tip bolus 322. The coil section 346 is formed after tube extrusion by the use of a jig that forms and holds the coil shape in a conventional manner while heat is applied.

The purpose of the coil section 346 is to provide a larger diameter end section on the tube that is, nevertheless, very flexible and resilient. Peristalsis exhibited by the jejunum can then hold the tip bolus 322 more easily. According to the invention, however, the coil section 336 is uncoiled, and straightened by the stylet and the guide wire before it is passed from the stomach, through the pyloric valve and into the duodenum. The passage 329 in the nose 328 of the tip bolus 322 for the wire 314 is shown.

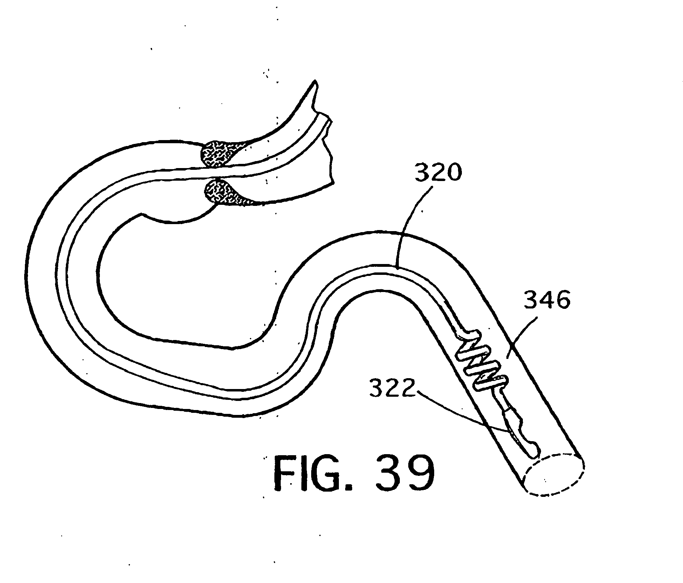

Referring now to FIG. 39, when the guide wire and stylet are removed, with the bolus 322 and the straightened coil section 346 in the jejunum, the unrestricted coil section automatically assumes its helical form. The tube 320 and tip bolus 322 then cannot be inadvertently pulled out past the Ligament of Trietz.

Thus, the tube 320 remains uncoiled and straight as long as either the stylet or the guide wire are in place, i.e., until the tip bolus 322 has entered the jejunum. Then the short coil section 336 is permitted to resume its normal shape. Enhanced retention force results.

While preferred embodiments of the invention have been described, it should be understood that the invention is not so limited and modifications may be made without departing from the invention. The scope of the invention is defined by the appended claims, and all devices that come within the meaning of the claims, by literally are intended to be embraced therein.

## Claims

1. A catheter insertion assembly, comprising:
a) a catheter tube (320) having a proximal end and a distal end,
b) a stylet (332) extending into said tube from said proximal end and moveable in said tube relative to said tube; and
c) said stylet (332) being movable longitudinally of said tube and a guide wire (314) to adjust the stiffness of said tube **characterised in that**
said catheter tube including a coiled section (346) adjacent said proximal end and a bolus on said distal end;
the guide wire (314) extending into said proximal end of said tube and out of said tube from said distal end, said tube being moveable along said guidewire relatively to said guidewire.

2. The catheter insertion assembly of Claim 1 further **characterized by** and including:
a) said guide wire (314) extending out of said bolus.

3. The catheter insertion assembly of Claim 1 further **characterized in that**:
a) said catheter tube includes a proximal section containing at least two lumens.

4. The catheter insertion assembly of Claim 2 further **characterized in that**:
a) said bolus (322) has a bullet nose; and
b) an opening (329) through said bullet nose through which said guide wire (314) extends.

5. The catheter insertion assembly of Claims 2 or 4 further **characterized in that**:
a) said normally coiled section (346) is adjacent said bolus (322);
b) said coiled section (346) being uncoiled by moving said catheter tube along said guidewire (314) so that said normally coiled section (346) moves along said guidewire (314).

6. The catheter insertion assembly of Claims 2 or 4 further **characterized in that**:
a) said bolus (322) has a bullet nose and a port on the side of said bolus; and
b) an aperture (329) in said nose through which said guide wire (314) passes.

7. The catheter insertion assembly of Claim 3 further **characterized in that**:
a) said catheter tube comprises a multiple lumen tube at its proximal end and a single lumen tube at its distal end, the tubes being interconnnected so that guide wire can extend the full length of both tubes.

## Patentansprüche

1. Kathetereinführanordnung, umfassend:
a) eine Katheterröhre (320), die ein proximales und ein distales Ende aufweist,
b) ein Stilett (332), das sich von dem proximalen Ende in die Röhre erstreckt und in der Röhre relativ zu der Röhre beweglich ist; und
c) wobei das Stilett (332) longitudinal von der Röhre und einem Führungsdraht (314) beweglich ist, um die Steifigkeit der Röhre anzupassen, **dadurch gekennzeichnet, dass**
die Katheterröhre einen gewickelten Abschnitt (346), der dem proximalen Ende benachbart ist, und einen Bolus an dem distalen Ende umfasst;
und wobei sich der Führungsdraht (314) in das proximale Ende der Röhre und von dem distalen Ende aus der Röhre heraus erstreckt, wobei die Röhre entlang des Führungsdrahts relativ zu dem Führungsdraht beweglich ist.

2. Kathetereinführanordnung nach Anspruch 1, ferner **dadurch gekennzeichnet, dass** und umfassend:
a) sich der Führungsdraht (314) aus dem Bolus heraus erstreckt.

3. Kathetereinführanordnung nach Anspruch 1, ferner **dadurch gekennzeichnet, dass**:
a) die Katheterröhre einen proximalen Abschnitt umfasst, der wenigstens zwei Lumina enthält.

4. Kathetereinführanordnung nach Anspruch 2, ferner **dadurch gekennzeichnet, dass**:
a) der Bolus (322) eine Kugelnase (engl.: bullet nose) aufweist; und
b) eine Öffnung (329) durch die Kugelnase, durch die sich der Führungsdraht (314) erstreckt.

5. Kathetereinführanordnung nach Anspruch 2 oder 4, ferner **dadurch gekennzeichnet, dass**:
a) der normal gewickelte Abschnitt (346) dem Bolus (322) benachbart ist;
b) der gewickelte Abschnitt (346) durch Bewegung der Katheterröhre entlang des Führungsdrahts (314), so dass sich der normal gewickelte Abschnitt (346) entlang des Führungsdrahts (314) bewegt, abgewickelt wird.

6. Kathetereinführanordnung nach Anspruch 2 oder 4, ferner **dadurch gekennzeichnet, dass**:
a) der Bolus (322) eine Kugelnase und einen Anschluss auf der Seite des Bolus' aufweist; und
b) eine Öffnung (329) in der Nase, durch die der Führungsdraht (314) verläuft.

7. Kathetereinführanordnung nach Anspruch 3, ferner **dadurch gekennzeichnet, dass**:
a) die Katheterröhre eine Mehrfachlumenröhre an ihrem proximalen Ende und eine Einfachlumenröhre an ihrem distalen Ende umfasst, wobei die Röhren miteinander verbunden sind, so dass der Führungsdraht sich entlang der ganzen Länge beider Röhren erstrecken kann.

## Revendications

1. Ensemble pour insertion de cathéter, comprenant :
a) un tube de cathéter (320) ayant une extrémité proximale et une extrémité distale ;
b) un stylet (332) s'étendant dans ledit tube à partir de ladite extrémité proximale et déplaçable dans ledit tube par rapport audit tube ; et
c) ledit stylet (332) étant déplaçable longitudinalement par rapport audit tube et un fil de guidage (314) pour régler la raideur dudit tube, **caractérisé en ce que**
ledit tube de cathéter comportant une section spiralée (346) adjacente à ladite extrémité proximale et un bolus sur ladite extrémité distale ;
le fil de guidage (314) s'étendant dans ladite extrémité proximale dudit tube et sortant dudit tube à partir de ladite extrémité distale, ledit tube étant déplaçable le long dudit fil de guidage par rapport audit fil de guidage.

2. Ensemble pour insertion de cathéter de la revendication 1 **caractérisé en outre par** et comportant :
a) ledit fil de guidage (314) s'étendant hors dudit bolus.

3. Ensemble pour insertion de cathéter de la revendication 1 **caractérisé en outre en ce que** :
a) ledit tube de cathéter comporte une section proximale contenant au moins deux lumières.

4. Ensemble pour insertion de cathéter de la revendication 2 **caractérisé en outre en ce que** :
a) ledit bolus (322) possède un nez en pointe ; et
b) une ouverture (329) à travers ledit nez en pointe à travers laquelle s'étend ledit fil de guidage (314).

5. Ensemble pour insertion de cathéter des revendications 2 ou 4 **caractérisé en outre en ce que** :
a) ladite section normalement spiralée (346) est adjacente audit bolus (322) ;
b) ladite section spiralée (346) étant déroulée en déplaçant ledit tube de cathéter le long dudit fil de guidage (314) de sorte que ladite section normalement spiralée (346) se déplace le long dudit fil de guidage (314).

6. Ensemble pour insertion de cathéter des revendications 2 ou 4 **caractérisé en outre en ce que** :
a) ledit bolus (322) possède un nez en pointe et un orifice sur le côté dudit bolus ; et
b) une ouverture (329) dans ledit nez à travers laquelle passe ledit fil de guidage (314).

7. Ensemble pour insertion de cathéter de la revendication 3 **caractérisé en outre en ce que** :
a) ledit tube de cathéter comprend un tube à plusieurs lumières au niveau de son extrémité proximale et un tube à une seule lumière au niveau de son extrémité distale, les tubes étant interconnectés de sorte que le fil de guidage peut s'étendre sur toute la longueur des deux tubes.
